(19) Europäisches Patentamt
European Patent Office
Office européen des brevets

(11) **EP 4 332 819 A1**

(12) **EUROPEAN PATENT APPLICATION**

(43) Date of publication:
**06.03.2024 Bulletin 2024/10**

(21) Application number: **23178787.0**

(22) Date of filing: **12.06.2023**

(51) International Patent Classification (IPC):
**G06F 30/15** (2020.01)    **G06F 30/23** (2020.01)
**G06F 113/26** (2020.01)    **G06F 111/06** (2020.01)

(52) Cooperative Patent Classification (CPC):
**G06F 30/15; G06F 30/23;** G06F 2111/06;
G06F 2113/26

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **29.08.2022 US 202217897999**

(71) Applicant: **The Boeing Company
Arlington, VA 22202 (US)**

(72) Inventors:
• **SABINO, John-Paul N.
Arlington, 22202 (US)**
• **RASHIDIAN, Bijan
Arlington, 22202 (US)**
• **DECO, Alberto
Arlington, 22202 (US)**
• **BHATIA, Kumar G.
Arlington, 22202 (US)**

(74) Representative: **Arnold & Siedsma
Bezuidenhoutseweg 57
2594 AC The Hague (NL)**

(54) **APPLYING STIFFNESS CHARACTERISTICS TO A COMPOSITE WING BOX FINITE ELEMENT MODEL**

(57)    Techniques for vehicle design are described. These techniques include receiving data relating to one or more desired stiffness characteristics for a manufactured component (302) and identifying a plurality of cross-sectional cuts for a model relating to the component (304). The techniques further include determining one or more material properties for the manufactured component, the one or more material properties estimated to meet the one or more desired stiffness characteristics for the manufactured component (314). This includes: for each cross-sectional cut, of the plurality of cross-sectional cuts: determining at least one of the one or more material properties based on identifying a potential solution to a nonlinear optimization problem using one or more initial estimates based on the desired stiffness characteristics.

300

Start

302 — Receive input data

304 — Create variables for each element that is intersected by a cut

306 — Create a solved/unsolved flag for each variable

308 — Create a canonical laminate property

310 — Initialize moduli of elasticity and shear moduli

312 — Interpolate specified stiffness characteristics to the stations of the cuts

314 — Solve each cut

End

**FIG. 3**

EP 4 332 819 A1

**Description**

**INTRODUCTION**

Aspects of the present disclosure relate to vehicle design and manufacture, and more specifically to aircraft wing design and manufacture.

Design of aerospace vehicles (e.g., aircraft) is extremely complex. For example, a finite element model can be used to determine stiffness characteristics for an aircraft wing box (e.g., a composite wing box) given properties of elements in the model (e.g., skin, spar, and stringer properties for the wing box). This process is, itself, quite complex. But determining the inverse, computing the properties of a finite element model that will result in the model having specified stiffness characteristics, is even more complex and can be effectively unsolvable using existing techniques. For example, improved techniques are needed to compute any, or all, of the skin, spar, or stringer properties of a composite wing box, given specified stiffness characteristics (e.g., any, or all, of vertical bending, chordwise bending, and torsional stiffness characteristics).

**BRIEF SUMMARY**

Embodiments include a method. The method includes receiving data relating to one or more desired stiffness characteristics for a manufactured component. The method further includes identifying a plurality of cross-sectional cuts for a model relating to the component. The method further includes determining one or more material properties for the manufactured component, the one or more material properties estimated to meet the one or more desired stiffness characteristics for the manufactured component. This includes: for each cross-sectional cut, of the plurality of cross-sectional cuts: determining at least one of the one or more material properties based on identifying a potential solution to a nonlinear optimization problem using one or more initial estimates based on the desired stiffness characteristics.

Embodiments further include a non-transitory computer-readable medium containing computer program code that, when executed by operation of one or more computer processors, performs operations. The operations include receiving data relating to one or more desired stiffness characteristics for a manufactured component. The operations further include identifying a plurality of cross-sectional cuts for a model relating to the component. The operations further include determining one or more material properties for the manufactured component, the one or more material properties estimated to meet the one or more desired stiffness characteristics for the manufactured component. This includes: for each cross-sectional cut, of the plurality of cross-sectional cuts: determining at least one of the one or more material properties based on identifying a potential solution to a nonlinear optimization problem using one or more initial estimates based on the desired stiffness characteristics.

Embodiments further include a system, including a computer processor and a memory having instructions stored thereon which, when executed on the computer processor, performs operations. The operations include receiving data relating to one or more desired stiffness characteristics for a manufactured component. The operations further include identifying a plurality of cross-sectional cuts for a model relating to the component. The operations further include determining one or more material properties for the manufactured component, the one or more material properties estimated to meet the one or more desired stiffness characteristics for the manufactured component. This includes: for each cross-sectional cut, of the plurality of cross-sectional cuts: determining at least one of the one or more material properties based on identifying a potential solution to a nonlinear optimization problem using one or more initial estimates based on the desired stiffness characteristics.

The following description and the related drawings set forth in detail certain illustrative features of one or more embodiments.

**BRIEF DESCRIPTION OF THE DRAWINGS**

The appended figures depict certain aspects of the one or more embodiments and are therefore not to be considered limiting of the scope of this disclosure.

FIG. 1 illustrates a manufacturing environment for applying bending and stiffness characteristics to a composite wing box finite element model, according to one embodiment.

FIG. 2 is a block diagram illustrating a manufacturing controller for applying bending and stiffness characteristics to a composite wing box finite element model, according to one embodiment.

FIG. 3 is a flowchart illustrating applying bending and stiffness characteristics to a composite wing box finite element model, according to one embodiment.

FIG. 4 illustrates desired stiffness characteristics for applying bending and stiffness characteristics to a composite wing box finite element model, according to one embodiment.

**FIG. 5** illustrates cuts defining cross sections of a finite element mode for applying bending and stiffness characteristics to a composite wing box finite element model, according to one embodiment.

**FIG. 6** is a flowchart illustrating solving cuts for applying bending and stiffness characteristics to a composite wing box finite element model, according to one embodiment.

**FIG. 7** illustrates variables solved per cut for applying bending and stiffness characteristics to a composite wing box finite element model, according to one embodiment.

**FIG. 8** illustrates solved differences relative to specified values for applying bending and stiffness characteristics to a composite wing box finite element model, according to one embodiment.

To facilitate understanding, identical reference numerals have been used, where possible, to designate identical elements that are common to the drawings. It is contemplated that elements and features of one embodiment may be beneficially incorporated in other embodiments without further recitation.

## DETAILED DESCRIPTION

In the following description, details are set forth by way of example to facilitate an understanding of the disclosed subject matter. It should be apparent to a person of ordinary skill in the field, however, that the disclosed implementations are exemplary and not exhaustive of all possible implementations. Thus, it should be understood that reference to the described examples is not intended to limit the scope of the disclosure. Any alterations and further modifications to the described devices, instruments, methods, and any further application of the principles of the present disclosure are fully contemplated as would normally occur to one skilled in the art to which the disclosure relates. In particular, it is fully contemplated that the features, components, and/or steps described with respect to one implementation may be combined with the features, components, and/or steps described with respect to other implementations of the present disclosure.

Embodiments of the present disclosure provide apparatuses, methods, processing systems, and computer-readable mediums for applying bending and stiffness characteristics to a class of wing box finite element models that are commonly used in aerospace in both product development and detailed design. In an embodiment, these techniques can be used in loads-stiffness convergence cycles and in trade studies, among other applications for aerospace development and design.

A wing of an aircraft can have numerous stiffness characteristics. These can include vertical bending stiffness (EIyy), chordwise bending stiffness (EIzz), and torsional stiffness (GJ). These stiffness characteristics are commonly dominant factors in the determination of the distributions of critical external loads (e.g., shear, moment, and torsion) for the wing. A wing box for an aircraft typically refers to the primary load-carrying structure of the wing. For a composite wing box (e.g., a wing box made of composite materials) that is built of skin, stringer, and spar idealizations, a sequence of load-stiffness convergence iterations are typically required to compute the true loads on a wing. This is because the stiffness impacts the loads, and the loads impact the stiffness, so iterations are required to reach a set of stable loads and stiffness distributions. While the external load envelopes ultimately depend on the precise distribution of the stiffness among the individual skin, spar, and stringer elements, the dependency is generally a secondary effect as compared to the dependency of those envelopes on the macroscopic stiffness characteristics (e.g., measurements of the cumulative stiffness of individual elements).

These loads-stiffness iterations can be very expensive to carry out (e.g., expensive both computationally and in terms of required development manpower). For example, once a finite element model of the wing is developed (e.g., with stiffnesses for its elements), typically one group of engineers will be responsible for generating the external loads for that model, and another group of engineers will be responsible for updating the stiffness of that structure to be airworthy in the presence of those loads. Both parts of this process can take several engineers significant time to carry out (e.g., a few weeks each), and both parts will usually need to be repeated several times until convergence is achieved.

In an embodiment, the loads-stiffness convergence cycle can be accelerated when the initial stiffness is a good approximation of the final converged stiffness. In some contexts, a good approximation of the final converged stiffness can be obtained from historical data, such as from a similar aircraft, including similar aircraft within the same airplane family. But this is only suitable where such historical data is available and applicable. In other contexts, a trade study of potential wing designs (e.g., a wing sweep study) can be performed, and the converged stiffness at a nominal sweep is available to seed the loads-stiffness convergence cycle for the variations.

Further, in an embodiment, EIyy, EIzz, and GJ can be calculated, given the skin and spar thicknesses and the stringer areas in addition to connectivity and material properties, using existing techniques. This procedure can be referred to as the forward problem. For example, existing techniques can be used to calculate EIyy, EIzz, and GJ, given the skin and spar thicknesses, and the stringer areas, in addition to connectivity and material properties. But these existing techniques cannot be used for what is referred to herein as the inverse problem: the estimation of the skin and spar thicknesses and stringer areas, given the EIyy, EIzz, and GJ. The forward problem can be used to validate a potential solution to the inverse problem (e.g., to compare the estimate to the desired stiffness distributions after mathematical modeling assumptions for the purpose of numerical analysis and solution of the inverse problem have been removed and the solution has been applied to the input finite element model), but techniques to solve the forward problem cannot be used to solve the inverse problem.

One or more techniques described herein estimate solutions to the inverse problem by setting values for the properties (i.e., thickness and areas) of the skin, stringer, and spar elements in the wing box structure so the resulting vertical bending stiffness, chordwise bending stiffness, and torsional stiffness of the box structure closely approximate given distributions of those three quantities across the span of the box. Once achieved, the resulting properties will allow for a useful approximation of the properties that would be obtained by a much more expensive external loads and structural sizing convergence iteration. That approximate estimate may be useful in a number of contexts, including as an initial guess for such a convergence iteration. An improved initial guess may result in fewer iterations required for convergence due to a superior approximation of the final stiffnesses.

In other words, one or more techniques described herein solve the problem of applying specified vertical bending, chordwise bending, and torsional stiffness distributions to a composite finite element wing box model. While no known existing solution to this problem exists, one potential approach to solving this problem would begin with creating a variable for each skin and spar ply direction and a variable for each stringer rod. A process to update the finite element model based on those variables would then be developed. Once an updated model was available, the model would be processed through a procedure that could calculate the stiffness characteristics for the model. That process could be used with a numerical optimization method, serving as a black box from which an objective and possibly constraints would be evaluated. The optimization method would determine the values of the variables. While this approach is relatively straightforward, it is extremely computationally expensive and poor in performance. This is due to the large number of design variables (typically several thousand variables) and the highly nonlinear nature of the stiffness calculations with respect to those design variables.

Further, a potential approach like the one described above may also use variables whose values are integer multiples of a single ply thickness in an attempt to model the discrete thicknesses achievable via the use of composite tape in manufacturing. These discrete variables for the skin and spar ply directions would be mixed with continuous variables for the stringer rod areas, resulting in a far more complex and difficult problem.

One or more embodiments described below improve on this by applying modeling assumptions to remove nonlinearities and decouple the variables into multiple smaller, more tractable, optimization problems (each typically having about 30-100 design variables). This reduces the typical time to full solution for all variables to tens of seconds (e.g., 30 seconds).

For example, the process to estimate solutions to the inverse problem can be characterized as a nonlinear least squares optimization problem with bound constraints on the variables. This can allow for reliable solutions to the optimization problem, using available tools. Further, in an embodiment, modeling simplifications that decouple variables and remove nonlinearities from the objective function can be used. This modeling solution can significantly improve the speed of the process, and reduce the computational expense. Caching of computed values that provide computational performance that result in an efficient solution sequence can also be done, in an embodiment. This can further reduce the computational cost of each iteration.

The disclosure further comprises the illustrative, examples set out in the following clauses:

Clause 1: A method, comprising: receiving data relating to one or more desired stiffness characteristics for a manufactured component; identifying a plurality of cross-sectional cuts for a model relating to the component; and determining one or more material properties for the manufactured component, the one or more material properties estimated to meet the one or more desired stiffness characteristics for the manufactured component, comprising: for each cross-sectional cut, of the plurality of cross-sectional cuts: determining at least one of the one or more material properties based on identifying a potential solution to a nonlinear optimization problem using one or more initial estimates based on the desired stiffness characteristics.

Clause 2: The method of clause 1, wherein: the manufactured component comprises an aircraft wing, the one or more desired stiffness characteristics comprise at least one of: (i) a vertical bending stiffness, (ii) a chordwise bending stiffness, or (iii) a torsional stiffness, relating to the aircraft wing, the one or more material properties comprise at least one of: (i) a thickness or (ii) a cross-sectional area for one or more elements of the aircraft wing, and the nonlinear optimization problem comprises a nonlinear least squares optimization problem.

Clause 3: The method of clause 2, wherein: the one or more desired stiffness characteristics comprise all of: (i) a vertical bending stiffness, (ii) a chordwise bending stiffness, and (iii) a torsional stiffness, relating to the aircraft wing, and the one or more material properties comprise both: (i) a ply thickness relating to at least one of skin or a spar for the aircraft wing, and (ii) a cross-sectional area of a stringer for the aircraft wing.

Clause 4: The method of any one of clauses 1 to 3, further comprising: creating a variable for a plurality of elements of the model intersected by at least one of the plurality of cross-sectional cuts, wherein the determining the one or more material properties comprises, for each cross-sectional cut, comprises: identifying one or more of the created variables associated with the cross-sectional cut; and determining the one or more initial estimates for the one or more variables based on the desired stiffness characteristics.

Clause 5: The method of clause 4, wherein each of the variables for the plurality of elements is a continuous variable.

Clause 6: The method of clause 4, further comprising: creating a boolean flag relating to each of the variables for the plurality of elements; and toggling a value for one or more of the boolean flags based on identifying the potential solution to the nonlinear optimization problem using the one or more initial estimates, for a given cross-sectional cut of the plurality of cross-sectional cuts.

Clause 7: The method of any one of clauses 1 to 6, further comprising interpolating at least some of the desired stiffness characteristics to a plurality of points for each of the plurality of cross-sectional cuts.

Clause 8: The method of any one of clauses 1 to 7, wherein: the one or more material properties relate to one or more elements of the manufactured component, and the model is generated to remove one or more nonlinearities relating to the determining the one or more material properties, the removed one or more nonlinearities relating to at least one of: (i) uniformity in cross-sectional properties for the one or more elements, (ii) spatial location relative to thickness for the one or more elements, (iii) spatial location relative to area for the one or more elements, (iv) or a constant ply percentage for a composite relating to the one or more elements of the manufactured component.

Clause 9: The method of any one of clauses 1 to 8, further comprising generating a canonical laminate property relating to one or more elements of the manufactured component.

Clause 10: The method of clause 9, further comprising determining at least one of: (i) a modulus of elasticity for the one or more elements, or (ii) a shear modulus for the one or more elements, based on the generated canonical laminate property.

Clause 11: A non-transitory computer-readable medium containing computer program code that, when executed by operation of one or more computer processors, performs operations comprising: receiving data relating to one or more desired stiffness characteristics for a manufactured component; identifying a plurality of cross-sectional cuts for a model relating to the component; and determining one or more material properties for the manufactured component, the one or more material properties estimated to meet the one or more desired stiffness characteristics for the manufactured component, comprising: for each cross-sectional cut, of the plurality of cross-sectional cuts: determining at least one of the one or more material properties based on identifying a potential solution to a nonlinear optimization problem using one or more initial estimates based on the desired stiffness characteristics.

Clause 12: The non-transitory computer-readable medium of clause 11, wherein: the manufactured component comprises an aircraft wing, the one or more desired stiffness characteristics comprise at least one of: (i) a vertical bending stiffness, (ii) a chordwise bending stiffness, or (iii) a torsional stiffness, relating to the aircraft wing, the one or more material properties comprise at least one of: (i) a thickness or (ii) a cross-sectional area for one or more elements of the aircraft wing, and the nonlinear optimization problem comprises a nonlinear least squares optimization problem.

Clause 13: The non-transitory computer-readable medium of clause 12, wherein:
the one or more desired stiffness characteristics comprise all of: (i) a vertical bending stiffness, (ii) a chordwise bending stiffness, and (iii) a torsional stiffness, relating to the aircraft wing, and the one or more material properties comprise both: (i) a ply thickness relating to at least one of skin or a spar for the aircraft wing, and (ii) a cross-sectional area of a stringer for the aircraft wing.

Clause 14: The non-transitory computer-readable medium of any one of clauses 11 to 13, the operations further comprising: creating a variable for a plurality of elements of the model intersected by at least one of the plurality of cross-sectional cuts, wherein the determining the one or more material properties comprises, for each cross-sectional cut, comprises: identifying one or more of the created variables associated with the cross-sectional cut; and determining the one or more initial estimates for the one or more variables based on the desired stiffness characteristics.

Clause 15: The non-transitory computer-readable medium of clause 14, the operations further comprising: creating a boolean flag relating to each of the variables for the plurality of elements; and toggling a value for one or more of the boolean flags based on identifying the potential solution to the nonlinear optimization problem using the one or more initial estimates, for a given cross-sectional cut of the plurality of cross-sectional cuts.

Clause 16: A system, comprising: a computer processor; and a memory having instructions stored thereon which, when executed on the computer processor, performs operations comprising: receiving data relating to one or more desired stiffness characteristics for a manufactured component; identifying a plurality of cross-sectional cuts for a model relating to the component; and determining one or more material properties for the manufactured component, the one or more material properties estimated to meet the one or more desired stiffness characteristics for the manufactured component, comprising: for each cross-sectional cut, of the plurality of cross-sectional cuts: determining at least one of the one or more material properties based on identifying a potential solution to a nonlinear optimization problem using one or more initial estimates based on the desired stiffness characteristics.

Clause 17: The system of clause 16, wherein: the manufactured component comprises an aircraft wing, the one or more desired stiffness characteristics comprise at least one of: (i) a vertical bending stiffness, (ii) a chordwise bending stiffness, or (iii) a torsional stiffness, relating to the aircraft wing, the one or more material properties comprise at least one of: (i) a thickness or (ii) a cross-sectional area for one or more elements of the aircraft wing, and the nonlinear optimization problem comprises a nonlinear least squares optimization problem.

Clause 18: The system of clause 17, wherein: the one or more desired stiffness characteristics comprise all of: (i) a vertical bending stiffness, (ii) a chordwise bending stiffness, and (iii) a torsional stiffness, relating to the aircraft wing, and the one or more material properties comprise both: (i) a ply thickness relating to at least one of skin or a spar for the aircraft wing, and (ii) a cross-sectional area of a stringer for the aircraft wing.

Clause 19: The system of any one of clauses 16 to 18, the operations further comprising: creating a variable for a plurality of elements of the model intersected by at least one of the plurality of cross-sectional cuts, wherein the determining the one or more material properties comprises, for each cross-sectional cut, comprises: identifying one or more of the created variables associated with the cross-sectional cut; and determining the one or more initial estimates for the one or more variables based on the desired stiffness characteristics.

Clause 20: The system of clause 19, the operations further comprising: creating a boolean flag relating to each of the variables for the plurality of elements; and toggling a value for one or more of the boolean flags based on identifying the potential solution to the nonlinear optimization problem using the one or more initial estimates, for a given cross-sectional cut of the plurality of cross-sectional cuts.

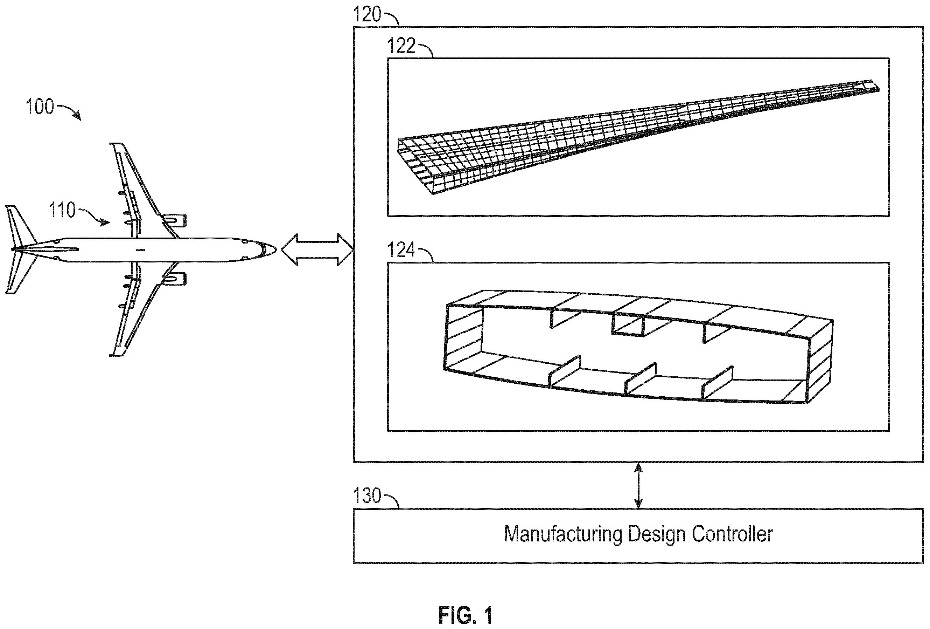

**FIG. 1** illustrates a manufacturing environment 100 for applying bending and stiffness characteristics to a composite wing box finite element model, according to one embodiment. In an embodiment, an aircraft 110 is designed using a manufacturing design controller 130. The manufacturing design controller uses a wing box finite element model 120. The wing box finite element model 120 is illustrated using a three-dimensional finite element model view 122 and a cross-sectional finite element model view 124.

In an embodiment, the finite element model can be defined using a number of elements and properties. For example, the finite element model can include connectivity elements used to represent skin, stringer, and spar components of the wing box. The connectivity elements provide data regarding their spatial locations and local material coordinates. For example, a NASA Structural Analysis (NASTRAN) finite element analysis program can be used to for finite element analysis. In this example, CQUAD4 and CTRIA3 connectivity types can be used for skin and spar elements, and a CROD connectivity type can be used for the stringers. These are merely examples, and any suitable analysis program can be used (e.g., any suitable NASTRAN program or other program) and any suitable connectivity types can be used. For example, CBAR or CBEAM elements can be used for the stringers.

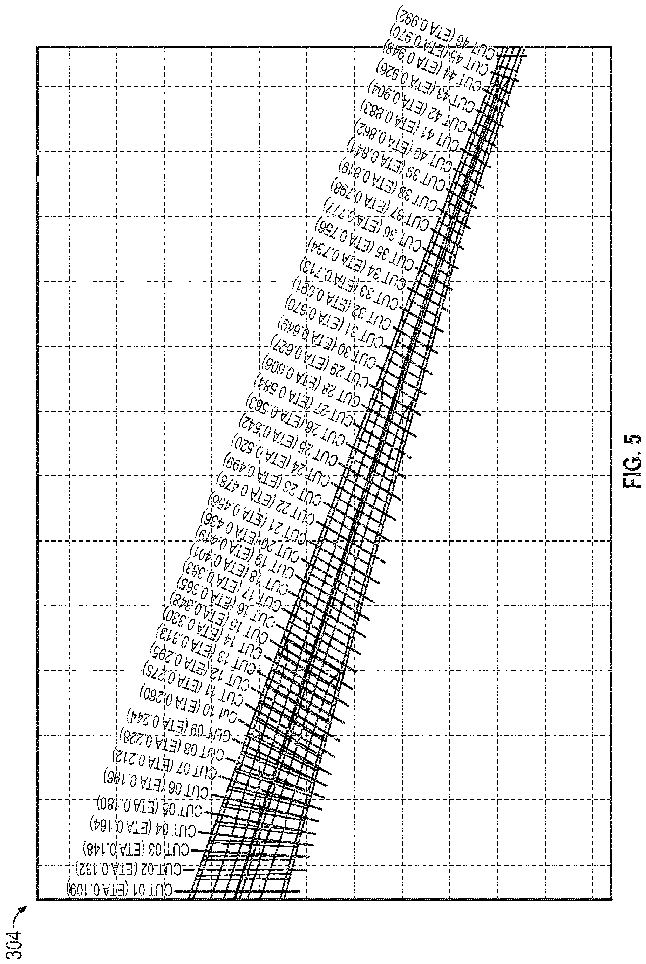

Further, in an embodiment, cross-sectional cuts can be computed for the target model. This is discussed further, below, with regard to **FIG. 5.** In an embodiment, the local stiffness values are computed for the cuts, and the cuts are specified as a list of local coordinate systems across the span of the wing box. Each local x-axis is typically inboard, with the y-axis aft and the z-axis up normal to a reference plane for the box. The origin of each cut should be in the interior of the cross-section of the box cut by the YZ-plane of the cut. With the axes arranged in such a manner, EIyy will refer to the vertical bending stiffness of the box and EIzz will refer to the chordwise bending stiffness. In NASTRAN, the CORD2R entry type can be used to represent these coordinate systems.

In an embodiment, the normalized "eta" station is calculated for each of the provided cuts. These stations are used to map the given stiffness distributions to the target model. In one embodiment, the same normalization process (e.g., dividing by the semi-span of the box) is used for both the input stiffness distributions and for the cuts on the target model. Alternatively, differing normalization processes can be used. For example, the normalization can result in an "eta" station of zero at the airplane centerline and an "eta" station of 1.0 at the outboard end of the box.

For the skin and spar elements of the wing box, a list of desired ply directions (e.g., 0, +45, -45, and 90 degrees) is defined relative to the local material axis system for each element. For each ply direction, a constant ply percentage of the overall element thickness (e.g., 40%, 25%, 25%, and 10%) can also be specified. Ply directions and ply percentages may be specified on an element-by-element basis, but often a single list of directions and ply percentages are used for the entire box.

Further, in an embodiment, for the skin and spar elements of the wing box composite material properties are defined in terms of the moduli of elasticity in the 0-degree and 90-degree fiber directions, Poisson's ratio, and in-plane shear modulus. Material density may also be provided (e.g., for the purposes of reporting the element weights). The material properties may be specified for each element, but typically the same material property is used for the entire box. In NASTRAN, these material properties are typically represented with the MAT8 material type.

For the stringer elements, the specified Young's modulus and shear modulus for the stringer material are defined. These values may be unique to each stringer element, or the same material property may be used for the entire set of stringer elements. Material density may be provided for the purposes of reporting the element weights. In NASTRAN, these material properties are typically represented with the MAT1 material type.

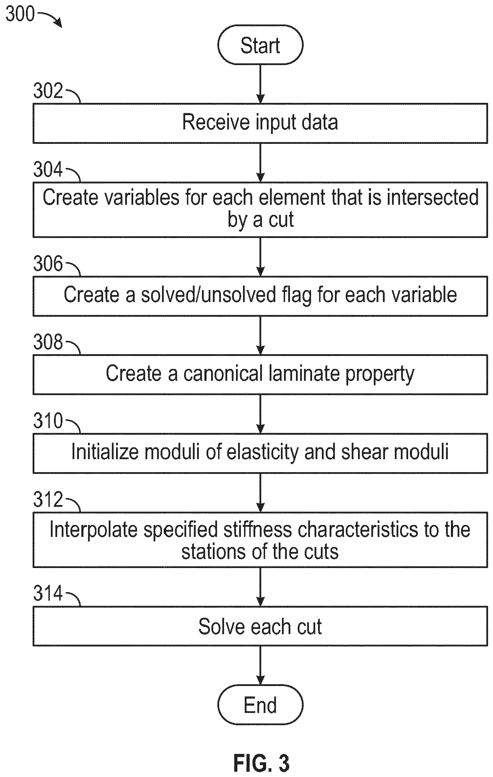

In an embodiment, the output of the techniques described below in relation to **FIG. 3** for estimating solutions to the inverse problem, described above, is a number of values. These output values include, for each skin and spar element cut by the input cutting planes, the ply thickness in each input direction. In NASTRAN, these thickness are typically represented by the PCOMP entry type. It further includes, for each stringer element cut by the input cutting planes, the cross-sectional area of the element. In NASTRAN, these thickness are typically represented by the PROD entry type if the stringer element was represented by a CROD connectivity entry type. This can also be generalized to PBAR and CBEAM. The output further incudes the resulting EIyy, EIzz, and GJ distributions for the wing box.

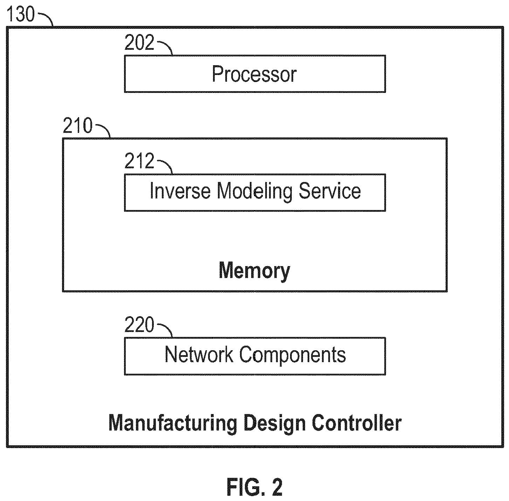

**FIG. 2** is a block diagram illustrating a manufacturing controller 130 for applying bending and stiffness characteristics to a composite wing box finite element model, according to one embodiment. In an embodiment, the manufacturing controller 130 includes a processor 202, a memory 210, and network components 220. The memory 210 may take the form of any non-transitory computer-readable medium. The processor 202 generally retrieves and executes programming instructions stored in the memory 210. The processor 202 is representative of a single central processing unit (CPU), multiple CPUs, a single CPU having multiple processing cores, graphics processing units (GPUs) having multiple execution paths, and the like.

The network components 220 include the components necessary for manufacturing controller 130 to interface with a suitable communication network. For example, the network components 220 can include wired, WiFi, or cellular network interface components and associated software. Although the memory 210 is shown as a single entity, the memory 210 may include one or more memory devices having blocks of memory associated with physical addresses, such as random access memory (RAM), read only memory (ROM), flash memory, or other types of volatile and/or non-volatile memory.

The memory 210 generally includes program code for performing various functions related to use of the manufacturing controller 130. The program code is generally described as various functional "applications" or "modules" within the memory 210, although alternate implementations may have different functions and/or combinations of functions. Within the memory 210, an inverse modeling service facilitates estimating solutions to the inverse problem, described above, for applying bending and stiffness characteristics to a composite wing box finite element model. This is described further, below, with regard to FIG. 3.

Although **FIG. 2** depicts the service 212 as located in the memory 210, that representation is merely provided as an illustration for clarity. More generally, the manufacturing design controller 130 may include one or more computing platforms, such as computer servers for example, which may be co-located, or may form an interactively linked but distributed system, such as a cloud-based system (e.g., a public cloud, a private cloud, a hybrid cloud, or any other suitable cloud-based system). As a result, the processor 202 and the memory 210 may correspond to distributed processor and memory resources within a computing environment. Thus, it is to be understood that the inverse modeling service 212 may be stored remotely from the manufacturing design controller 130 within the distributed memory resources of a computing environment.

**FIG. 3** is a flowchart 300 illustrating applying bending and stiffness characteristics to a composite wing box finite element model, according to one embodiment. In an embodiment, **FIG. 3** illustrates a technique to identify skin and spare web thickness values, and stringer rod areas, to achieve a desired set of stiffness distributions. This is referred to above as the inverse problem.

In an embodiment, the inverse problem is an underdetermined problem, meaning that it typically has more variables (e.g., areas and thicknesses) than constraints (e.g., match a given EIyy, EIzz, and GJ). This problem is also nonlinear in its variables. One or more assumptions and simplifications can be used develop tractable approximation of the inverse problem, which can be solved using the techniques illustrated in **FIG. 3.**

As a first example of an assumption that can be used, the cross-sectional properties of each skin or spar web (e.g., thickness and material properties) can be treated as uniform across their spatial extent. Similarly, each stringer rod can be modeled as a uniform disc in its cross section. Any differences in thickness can be ignored.

As another example of an assumption that can be used, the spatial locations of the elements (e.g., skin, spar, stringer) can be assumed to be independent of the thickness or area of the elements. In an embodiment, finite element models are often designed to place elements in locations relative to a reference outer mold line, and the location of an element will depend on its thickness. This assumption typically has a minor effect on the resulting stiffness profiles in realistic applications. Assuming that spatial locations are independent of thickness or area removes nonlinearities while having only a minor effect on the estimated solution, especially compared with the typical improvement of the stiffness profiles as a result of following the techniques illustrated in **FIG. 3.**

As another example of an assumption that can be used, the skin and spar elements can be assumed to be composite elements (e.g., in NASTRAN, PCOMPs) with pre-determined constant ply percentages (e.g., 10% 0-degree, 25% +45-degree, 25% -45-degree, and 10% 90-degree). This assumption allows for the creation of a single design variable (total thickness) for each web instead of one design variable (ply thickness) per ply direction per web. This also makes the moduli of elasticity and shear modulus for each element constant, which can be demonstrated using known techniques in composite laminate theory.

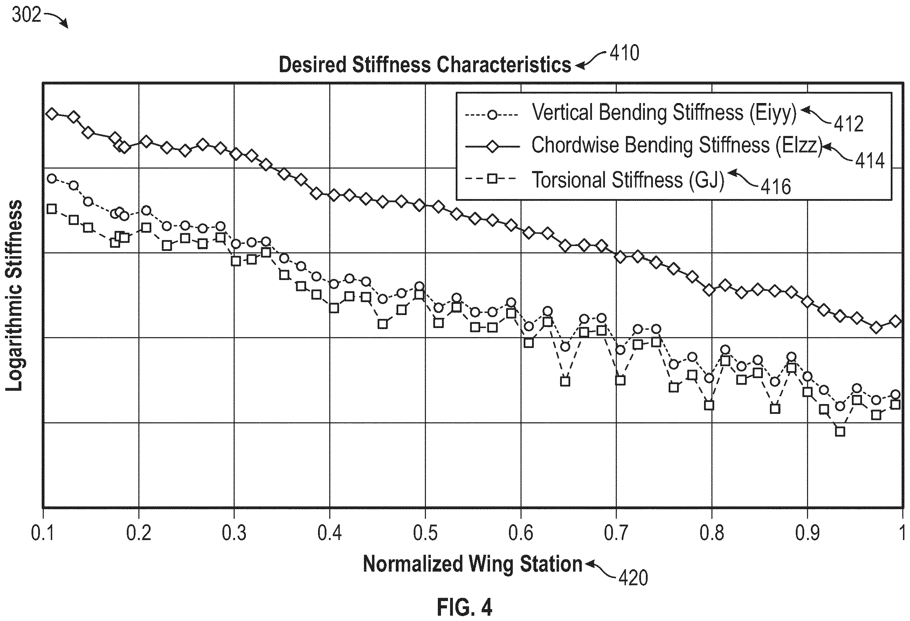

Applying these assumptions, an inverse modeling service (e.g., the inverse modeling service 212 illustrated in **FIG. 2**) can use the techniques illustrated in **FIG. 3** to identify skin and spare web thickness values, and stringer rod areas, to achieve a desired set of stiffness distributions. At block 302, the inverse modeling service receives input data. For example, the inverse modeling service can receive desired vertical bending stiffness (EIyy), desired chordwise bending stiffness (EIzz), and desired torsional stiffness (GJ). This is discussed further, below, with regard to **FIG. 4.**

At block 304, the inverse modeling service creates variables for each element that is intersected by a cut. For example, as discussed above, the inverse modeling service can compute cross-sectional cuts for the wing box model. This is discussed further, below, with regard to **FIG. 5.**

In an embodiment, the inverse modeling service creates a variable $x_i$ for each skin and spar element cut by any cutting plane. Each of these variables will represent the total thickness of the element and its value will be determined using the techniques illustrated in **FIG. 3.** The inverse modeling service can further create additional variables $x_i$ for each stringer element cut by a cutting plane. Each of these variables will represent the stringer rod area (e.g., perpendicular to its axis, which is not necessarily perpendicular to any cutting plane) and its value will be determined by the techniques illustrated in **FIG. 3.** For example, for the model 120 illustrated above in **FIG. 1,** and the cross sectional cuts illustrated below in **FIG. 5,** the inverse modeling service would create 57 total variables for the first cut, including 17 skin element thickness variables, 8 spar element thickness variables, 22 stringer rod area variables, and 10 spar chord rod area variables. In an embodiment the spar chord rod area variables are grouped with the stringer rod area variables.

In an embodiment each of these variables is a continuous variable. Alternatively, some (or all) of the variables are discrete variables. As discussed above, in an embodiment the use of only continuous variables reduces the computational complexity of the techniques illustrated in **FIG. 3.**

At block 306, the inverse modeling service creates a solved or unsolved flag for each variable (e.g., for each variable created at block 304). For example, the inverse modeling service can create a boolean flag (e.g., a true or false variable) to indicate whether each variable is solved or unsolved. In an embodiment, all of these boolean flags are initialized to false. Further, in an embodiment the inverse modeling service can initialize a mapping from the variables to the corresponding model elements, and vice-versa. For example, the inverse modeling service can establish the mapping using variable indices and element identification numbers.

At block 308, the inverse modeling service creates a canonical laminate property. For example, the inverse modeling service can create a one-inch thick canonical laminate property using the input values received at block 302. This property can be represented by an object that has available techniques to: (i) rotate the ply orientations counterclockwise by a given angle, (ii) scale the thicknesses of each ply to achieve a desired total thickness, (iii) compute the resulting moduli of elasticity of the scaled laminate (e.g., via composite laminate theory), and (iv) compute the resulting shear modulus of the scaled laminate (e.g., via composite laminate theory).

At block 310, the inverse modeling service initializes moduli of elasticity and shear moduli. In an embodiment, the inverse modeling service initialize the modulus of elasticity $E_i$ for each element in the model. For the stringer rods, these factors can be the Young's moduli of the corresponding material for each rod. For the skin and spar webs, these factors can be the modulus of elasticity in the zero-degree ply direction, $E_x$, for the laminate property obtained by rotating the canonical laminate property by the angle that the x-axis of the material coordinate system makes with the global x-axis. Due to the simplification that the ply orientations and ply percentages for each web will be invariant, discussed above, this factor can be computed from the canonical one-inch thick laminate property (e.g., created at block 308) after rotating it by that angle. Note that the webs will typically not have identical moduli of elasticity because their material axis systems typically are not identical. Similarly, in an embodiment the inverse modeling service initializes a shear

modulus $G_i$ for each web element. The inverse modeling service use a similar approach to initializing $E_i$ via the rotation of the canonical property, except that the shear modulus is calculated instead of the modulus of elasticity.

At block 312, the inverse modeling service interpolates specified stiffness characteristics to the stations of the cuts. In an embodiment, the inverse modeling service interpolates the specified EIyy, EIzz, and GJ values to the stations of the cuts. If the station for any cut is outside the bounds of the distribution for these values, then the inverse modeling service uses the value of the nearest station (e.g., use the value at 0.1 if a cut is located at 0.08 and the specified values were defined only between 0.1 and 1.0).

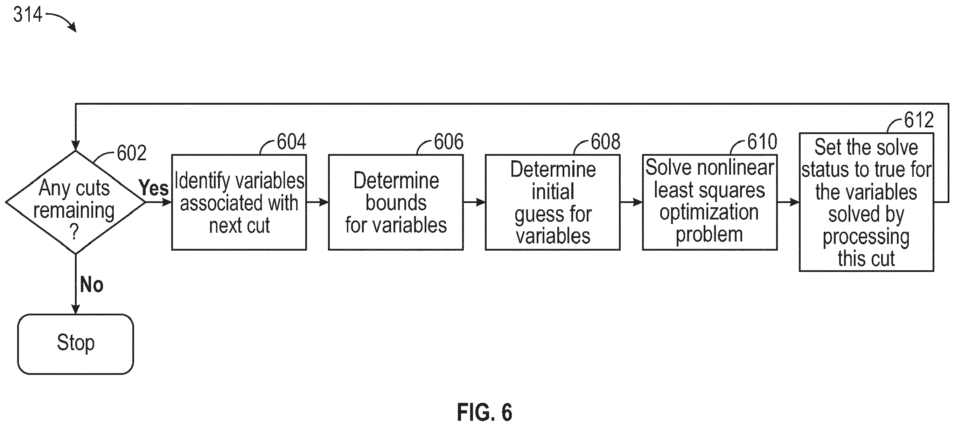

At block 314, the inverse modeling service solves for each cut. This is discussed further, below, with regard to **FIG. 6.** For example, the inverse modeling service can solve the cuts one-by-one using the techniques illustrated in **FIG. 6.**

**FIG. 4** illustrates desired stiffness characteristics 410 for applying bending and stiffness characteristics to a composite wing box finite element model, according to one embodiment. In an embodiment, **FIG. 4** illustrates example input values received by an inverse modeling service (e.g., the inverse modeling service 212 illustrated in **FIG. 2**), as discussed above in relation to block 302 illustrated in **FIG. 3.** In an embodiment, the desired stiffness characteristics 410 include desired vertical bending stiffness (EIyy) 412, desired chordwise bending stiffness (EIzz) 414, and desired torsional stiffness (GJ) 416. These are illustrated using a graph with a normalized wing station 420 across the x-axis and logarithmic stiffness across the y-axis.

**FIG. 5** illustrates cuts defining cross sections of a finite element mode for applying bending and stiffness characteristics to a composite wing box finite element model, according to one embodiment. In an embodiment, **FIG. 5** illustrates one example of cross-sectional cuts for a target model (e.g., with 46 cuts from Cut 01 to Cut 46). As discussed above in relation to block 304 illustrated in **FIG. 3,** in an embodiment local stiffness values are computed at the cuts. The cuts can be specified as a list local coordinate systems across the span of the box. Each local x-axis can be inboard, with the y-axis aft and the z-axis up normal to a reference plane for the box. The origin of each cut is the interior of the cross-section of the box cut by the YZ-plane of the cut. With the axes arranged in such a manner, EIyy will refer to the vertical bending stiffness of the box and EIzz will refer to the chordwise bending stiffness. In NASTRAN, the CORD2R entry type can be used to represent these coordinate systems.

**FIG. 6** is a flowchart illustrating solving cuts for applying bending and stiffness characteristics to a composite wing box finite element model, according to one embodiment. In an embodiment, **FIG. 6** corresponds with block 314 illustrated in **FIG. 3.** At block 602, an inverse modeling service (e.g., the inverse modeling service 212 illustrated in **FIG. 2**) determines whether any cuts are remaining. If not, the flow ends. If so, the flow proceeds to block 604.

At block 604, the inverse modeling service identifies variables associated with the next cut. In an embodiment, the inverse modeling service identifies which skin, spar, and stringer elements are intersected by the cutting plane for the cut. The inverse modeling service then partitions the elements into two categories: (i) the elements for which the associated variables have not yet been solved using another cut, and (ii) the elements for which the associated variables have already been solved using another cut. For example, as illustrated in **FIG. 5,** some of the variables for Cut 05 will likely have been solved when Cuts 03 and 04 were processed.

At block 606, the inverse modeling service determines bounds for the variables. In an embodiment, the bounds are independent of the element type and location, but the bounds can be set differently for each element. For example, a lower bound of 0.07 and an upper bound of 2.0 could be used. The skin and spar thickness variables have units of length (e.g., inches), but the stringer area variables have units of area (e.g., sq. inches).

At block 608, the inverse modeling service determines an initial guess for the variables (e.g., the variables identified at block 604). In an embodiment, the inverse modeling service constructs an objective function that measures the difference between the $EI_{yy}(x)$, $EI_{zz}(x)$, $GJ(x)$ values, for a given set of values of the variables x, from the target $EI_{yy}^*, EI_{zz}^*$, $GJ^*$ for the cut. If either one or two of EIyy, EIzz, and GJ are not desired to be matched, set the corresponding part of the objective to zero. This can be done using the equation below:

$$f = \frac{1}{2}\left(\left(\frac{EI_{yy}(x)}{EI_{yy}^*} - 1\right)^2 + \left(\frac{EI_{zz}(x)}{EI_{zz}^*} - 1\right)^2 + \left(\frac{GJ(x)}{GJ^*} - 1\right)^2\right)$$

If any of the target values is zero, the inverse modeling service can omit the corresponding part of the objective function to avoid the division by zero. For example, if $EI_{yy}^* = 0$, then the equation below can be used:

$$f = \frac{1}{2}\left(\left(\frac{EI_{zz}(x)}{EI_{zz}^*} - 1\right)^2 + \left(\frac{GJ(x)}{GJ^*} - 1\right)^2\right)$$

The normalization of each stiffness term results in an equal weighting of each term. Without the normalization (e.g., allowing a term to be $\left(EI_{yy}(x) - EI_{yy}^*\right)^2$ ), one of the terms may dominate the objective functions, possibly resulting a reasonable match for only that stiffness term but not for either of the other terms. The objective function is nonlinear in the variable $x$ , as is shown below in the details of the computation of the terms $EI_{yy}^*(x), EI_{zz}^*(x)$ , $GJ^*(x)$.

In an embodiment, the techniques discussed above can further be used for matching of an $EI_{yz}$ term. Further, a term for weight, center of gravity, or another characteristic could be included. But this is typically not used, because (a) the additional terms may detract from the ability of the numerical optimization to match the primary stiffness targets $EI_{yy}^*, EI_{zz}^*$ , $GJ^*$, and (b) the transference of weight or center of gravity from a reference model to the model of interest may be nonsensical. In limited cases (e.g., sensitivity studies based on a single set of geometry), it may be worth including one or more of these additional terms into the objective function.

Using the objective function, the inverse modeling service constructs an initial guess for the variables. For example, the inverse modeling service can create two auxiliary variables s and $t$ and an integer $N$. Allow s and $t$ to independently take on the values L + (J ÷ N) * (U - L), where N = 0, ... , N. For each pair of potential values (s, t), set the skin and spar thickness variables to s and the stringer rod area variables to t. Set the initial guess for the variables to the corresponding values (s*, t*) that minimize the objective function over these $(N + 1)^2$ candidates. A value of N = 5 can be used.

At block 610, the inverse modeling service solves a nonlinear least squares optimization problem. For example, the inverse modeling service can solve for a set of values x* that minimizes the objective function f (e.g., creates at block 608) over the domain of allowed variable values. In an embodiment, any suitable nonlinear least-squares solver can be applied to this problem. For example, MATLAB® or another available numerical nonlinear least-squares solver can be used (e.g., another programming language or mathematical or engineering tool).

In an embodiment, numerical derivatives are typically sufficient to solve the nonlinear least squares optimization problem, and an analytical gradient for the objective is not required. Without analytical gradients, the number of iterations for the numerical optimization may be high, but the cost per iteration is low due to the modeling decisions described above and judicious caching of repeatedly used values, such as the EA-weighted cut center of gravity for a given set of variable values, which is required in the computation of Iyy and Izz for each element in a cut. This is merely an example, and an analytical gradient can also be used.

At block 612, the inverse modeling service sets the solved status to True for the variables that have been solved by processing this cut. The flow then returns to block 602.

In an embodiment, the inverse modeling service further uses the solved variables to compute $EI_{yy}(x)$, $EI_{zz}(x)$, and $GJ(x)$. For example, the calculation of $EI_{yy}(x)$ and $EI_{zz}(x)$ are generally similar, distinguished only by changes in coordinates. Computing $EI_{yy}(x)$ can be used to explain both. For example, $EI_{yy}(x)$ can be computed using the equation below:

$$EI_{yy(x)} = \sum_{\substack{i \in \text{elements} \\ \text{in cut}}} E_i I_{y,i}(x)$$

In this equation, $I_{y,i}(x)$ is calculated as

$$\begin{cases} \dfrac{x_i^2}{4\pi} + \dfrac{x_i \delta_{z,i}^2(x)}{|cos\theta|} & \text{if element } i \text{ is a stringer rod} \\ \dfrac{x_i w^3}{12.0} + x_i w \delta_{z,i}^2(x) & \text{if element } i \text{ is a spar element} \\ \dfrac{x_i^3 w}{12.0} + x_i w \delta_{z,i}^2(x) & \text{if element } i \text{ is a skin element} \end{cases}$$

In an embodiment, some of the variables in this computation may have been previously solved when another cut was processed. Further, due to the modeling assumptions described above, $E_i$ does not depend on the variables. As above, $x_i/|cos\theta|$ is the cross-sectional area of the stringer rod in the plane of the cut when element $i$ is a stringer. For the skin and spar webs, w is the width of the element in the plane of the cut. In practice, web elements may not be explicitly identified as "skin" or "spar" elements, but their horizontal or vertical orientation is determined by their major axis orientation in the plane of the cut. The quantity $\delta_{z,i}(x)$ measures the distance between the z spatial coordinates of the element centroid and the EA-weighted centroid of all the elements in the box, as shown by the following equation:

$$\delta_{z,i}(x) = z_i - \frac{\sum_j E_j A_j(x_j) z_j}{\sum_j E_j A_j(x_j)}$$

Here $j$ is over all the elements intersected by the cut. Note that $\delta_{z,i}(x)$ is nonlinear in the variables $\boldsymbol{x}$ and depends upon all variables associated with the elements in the cut. As noted, $EI_{zz}(x)$ can be computed using a similar technique to the computation described above for $EI_{yy}(x)$.

In an embodiment, $GJ(x)$ is computed using the equation:

$$GJ(x) = \frac{4\,A_{\text{enclosed}}^2}{\sum_{i \in \text{webs}} \frac{w_i}{G_i\,x_i}}$$

The value $A_{\text{enclosed}}$, the area of the box intersected by the cut, can be computed as the sum of triangles around the cross-section of the cut. Note that the summation is only over the skin and spar webs intersected by the cut, and that the widths $w_i$ and shear moduli $G_i$ are constant with respect to the variables $x$ due to the modeling assumptions described above. Finally, $GJ(x)$ is nonlinear in the variables $x$.

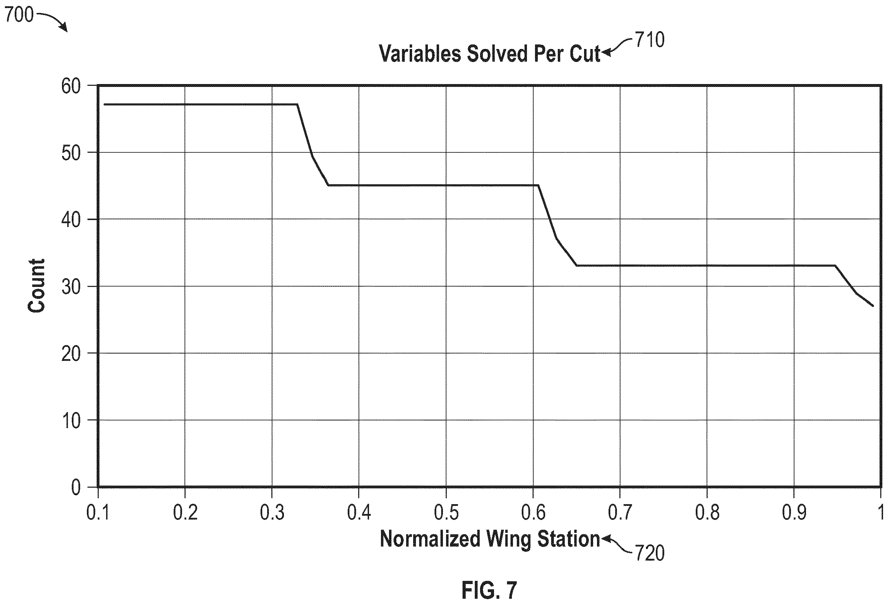

**FIG. 7** illustrates variables solved per cut 700 for applying bending and stiffness characteristics to a composite wing box finite element model, according to one embodiment. In an embodiment, solving for each cut (e.g., as discussed above in relation to **FIG. 6**) solves for multiple variables. These variables can apply to several cuts (e.g., solving for one cut can also solve variables associated with another cut). **FIG. 7** illustrates the count of variables solved per cut 710, along the y-axis, for each cut in the normalized wing station 720, along the x-axis (e.g., for the cuts illustrated in **FIG. 5**). In an embodiment, as the wing box becomes narrower, the number of variables solved by each cut decreases. For example, as illustrated a total of 2020 variables were determined. The variables were distributed among 46 cuts, with a mean of about 44 variables per cut (with a minimum of 27 and a maximum of 57 variables assigned to a cut).

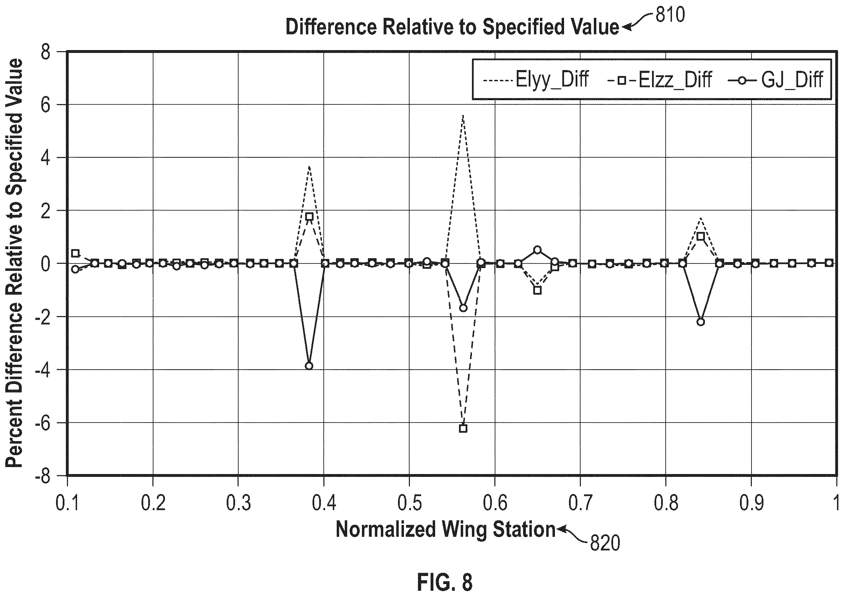

**FIG. 8** illustrates solved differences relative to specified values for applying bending and stiffness characteristics to a composite wing box finite element model, according to one embodiment. In an embodiment, **FIG. 8** illustrates the relative differences 810 between the solved stiffness characteristics generated using the techniques illustrated at **FIGS. 3-6,** and measured stiffness characteristics, for a normalized wing station 820. In an embodiment, the match was typically within 1%.

*Additional Considerations*

The preceding description is provided to enable any person skilled in the art to practice the various embodiments described herein. The examples discussed herein are not limiting of the scope, applicability, or embodiments set forth in the claims. Various modifications to these embodiments will be readily apparent to those skilled in the art, and the generic principles defined herein may be applied to other embodiments. For example, changes may be made in the function and arrangement of elements discussed without departing from the scope of the disclosure. Various examples may omit, substitute, or add various procedures or components as appropriate. For instance, the methods described may be performed in an order different from that described, and various steps may be added, omitted, or combined. Also, features described with respect to some examples may be combined in some other examples. For example, an apparatus may be implemented or a method may be practiced using any number of the aspects set forth herein. In addition, the scope of the disclosure is intended to cover such an apparatus or method that is practiced using other structure, functionality, or structure and functionality in addition to, or other than, the various aspects of the disclosure set forth herein. It should be understood that any aspect of the disclosure disclosed herein may be embodied by one or more elements of a claim.

As used herein, the word "exemplary" means "serving as an example, instance, or illustration." Any aspect described herein as "exemplary" is not necessarily to be construed as preferred or advantageous over other aspects.

As used herein, a phrase referring to "at least one of" a list of items refers to any combination of those items, including single members. As an example, "at least one of: a, b, or c" is intended to cover a, b, c, a-b, a-c, b-c, and a-b-c, as well as any combination with multiples of the same element (e.g., a-a, a-a-a, a-a-b, a-a-c, a-b-b, a-c-c, b-b, b-b-b, b-b-c, c-c, and c-c-c or any other ordering of a, b, and c).

As used herein, the term "determining" encompasses a wide variety of actions. For example, "determining" may include calculating, computing, processing, deriving, investigating, looking up (e.g., looking up in a table, a database or another data structure), ascertaining and the like. Also, "determining" may include receiving (e.g., receiving information), accessing (e.g., accessing data in a memory) and the like. Also, "determining" may include resolving, selecting, choosing, establishing and the like.

The methods disclosed herein comprise one or more steps or actions for achieving the methods. The method steps and/or actions may be interchanged with one another without departing from the scope of the claims. In other words, unless a specific order of steps or actions is specified, the order and/or use of specific steps and/or actions may be modified without departing from the scope of the claims. Further, the various operations of methods described above may be performed by any suitable means capable of performing the corresponding functions. The means may include various hardware and/or software component(s) and/or module(s), including, but not limited to a circuit, an application specific integrated circuit (ASIC), or processor. Generally, where there are operations illustrated in figures, those operations may have corresponding counterpart means-plus-function components with similar numbering.

The following claims are not intended to be limited to the embodiments shown herein, but are to be accorded the full scope consistent with the language of the claims. Within a claim, reference to an element in the singular is not intended to mean "one and only one" unless specifically so stated, but rather "one or more." Unless specifically stated otherwise, the term "some" refers to one or more. No claim element is to be construed under the provisions of 35 U.S.C. §112(f) unless the element is expressly recited using the phrase "means for" or, in the case of a method claim, the element is recited using the phrase "step for." All structural and functional equivalents to the elements of the various aspects described throughout this disclosure that are known or later come to be known to those of ordinary skill in the art are expressly incorporated herein by reference and are intended to be encompassed by the claims. Moreover, nothing disclosed herein is intended to be dedicated to the public regardless of whether such disclosure is explicitly recited in the claims.

## Claims

1. A method, comprising:

   receiving data relating to one or more desired stiffness characteristics for a manufactured component (302);
   identifying a plurality of cross-sectional cuts for a model relating to the component (304); and
   determining one or more material properties for the manufactured component, the one or more material properties estimated to meet the one or more desired stiffness characteristics for the manufactured component (314), comprising:
   for each cross-sectional cut, of the plurality of cross-sectional cuts:
   determining at least one of the one or more material properties based on identifying a potential solution to a nonlinear optimization problem using one or more initial estimates based on the desired stiffness characteristics (610).

2. The method of claim 1, wherein:

   the manufactured component comprises an aircraft wing (110),
   the one or more desired stiffness characteristics comprise at least one of: (i) a vertical bending stiffness (412), (ii) a chordwise bending stiffness (414), or (iii) a torsional stiffness (416), relating to the aircraft wing,
   the one or more material properties comprise at least one of: (i) a thickness or (ii) a cross-sectional area for one or more elements of the aircraft wing (314), and
   the nonlinear optimization problem comprises a nonlinear least squares optimization problem (610).

3. The method of claim 2, wherein:

   the one or more desired stiffness characteristics comprise all of: (i) a vertical bending stiffness (412), (ii) a chordwise bending stiffness (414), and (iii) a torsional stiffness (416), relating to the aircraft wing, and
   the one or more material properties comprise both: (i) a ply thickness relating to at least one of skin or a spar for the aircraft wing, and (ii) a cross-sectional area of a stringer for the aircraft wing (610).

4. The method of any of claims 1 to 3, further comprising:

creating a variable for a plurality of elements of the model intersected by at least one of the plurality of cross-sectional cuts (304),
wherein the determining the one or more material properties comprises, for each cross-sectional cut, comprises:

identifying one or more of the created variables associated with the cross-sectional cut (604); and
determining the one or more initial estimates for the one or more variables based on the desired stiffness characteristics (608).

5. The method of claim 4, wherein each of the variables for the plurality of elements is a continuous variable (606).

6. The method of claim 4, further comprising:

creating a boolean flag relating to each of the variables for the plurality of elements (306); and
toggling a value for one or more of the boolean flags based on identifying the potential solution to the nonlinear optimization problem using the one or more initial estimates, for a given cross-sectional cut of the plurality of cross-sectional cuts (612).

7. The method of any of claims 1 to 6, further comprising interpolating at least some of the desired stiffness characteristics to a plurality of points for each of the plurality of cross-sectional cuts (312).

8. The method of any of claims 1 to 7, wherein:

the one or more material properties relate to one or more elements of the manufactured component (110), and the model is generated to remove one or more nonlinearities relating to the determining the one or more material properties (300), the removed one or more nonlinearities relating to at least one of: (i) uniformity in cross-sectional properties for the one or more elements, (ii) spatial location relative to thickness for the one or more elements, (iii) spatial location relative to area for the one or more elements, (iv) or a constant ply percentage for a composite relating to the one or more elements of the manufactured component.

9. The method of any of claims 1 to 8, further comprising generating a canonical laminate property relating to one or more elements of the manufactured component (308).

10. The method of claim 9, further comprising determining at least one of: (i) a modulus of elasticity for the one or more elements, or (ii) a shear modulus for the one or more elements, based on the generated canonical laminate property (310).

11. A system, comprising:

a computer processor; and
a memory having instructions stored thereon which, when executed on the computer processor, performs operations comprising:

receiving data relating to one or more desired stiffness characteristics for a manufactured component (302);
identifying a plurality of cross-sectional cuts for a model relating to the component (304); and
determining one or more material properties for the manufactured component, the one or more material properties estimated to meet the one or more desired stiffness characteristics for the manufactured component (314), comprising:
for each cross-sectional cut, of the plurality of cross-sectional cuts:
determining at least one of the one or more material properties based on identifying a potential solution to a nonlinear optimization problem using one or more initial estimates based on the desired stiffness characteristics (610).

12. The system of claim 11, wherein:

the manufactured component comprises an aircraft wing (110),
the one or more desired stiffness characteristics comprise at least one of: (i) a vertical bending stiffness (412), (ii) a chordwise bending stiffness (414), or (iii) a torsional stiffness (416), relating to the aircraft wing,
the one or more material properties comprise at least one of: (i) a thickness or (ii) a cross-sectional area for one or more elements of the aircraft wing (314), and
the nonlinear optimization problem comprises a nonlinear least squares optimization problem (610).

13. The system of claim 12, wherein:

the one or more desired stiffness characteristics comprise all of: (i) a vertical bending stiffness (412), (ii) a chordwise bending stiffness (414), and (iii) a torsional stiffness (416), relating to the aircraft wing, and
the one or more material properties comprise both: (i) a ply thickness relating to at least one of skin or a spar for the aircraft wing, and (ii) a cross-sectional area of a stringer for the aircraft wing (610).

14. The system of any of claims 11 to 13, the operations further comprising:

creating a variable for a plurality of elements of the model intersected by at least one of the plurality of cross-sectional cuts (304),
wherein the determining the one or more material properties comprises, for each cross-sectional cut, comprises:

identifying one or more of the created variables associated with the cross-sectional cut (604); and
determining the one or more initial estimates for the one or more variables based on the desired stiffness characteristics (608).

15. The system of claim 14, the operations further comprising:

creating a boolean flag relating to each of the variables for the plurality of elements (306); and
toggling a value for one or more of the boolean flags based on identifying the potential solution to the nonlinear optimization problem using the one or more initial estimates, for a given cross-sectional cut of the plurality of cross-sectional cuts (612).

FIG. 1

FIG. 2

300

```
            ( Start )
                |
                v
302 ┌─────────────────────────────────────────┐
    │            Receive input data             │
    └─────────────────────────────────────────┘
                |
                v
304 ┌─────────────────────────────────────────┐
    │  Create variables for each element that   │
    │          is intersected by a cut          │
    └─────────────────────────────────────────┘
                |
                v
306 ┌─────────────────────────────────────────┐
    │  Create a solved/unsolved flag for each   │
    │                 variable                  │
    └─────────────────────────────────────────┘
                |
                v
308 ┌─────────────────────────────────────────┐
    │       Create a canonical laminate         │
    │                property                   │
    └─────────────────────────────────────────┘
                |
                v
310 ┌─────────────────────────────────────────┐
    │ Initialize moduli of elasticity and shear │
    │                 moduli                    │
    └─────────────────────────────────────────┘
                |
                v
312 ┌─────────────────────────────────────────┐
    │  Interpolate specified stiffness          │
    │  characteristics to the stations of the   │
    │                 cuts                      │
    └─────────────────────────────────────────┘
                |
                v
314 ┌─────────────────────────────────────────┐
    │               Solve each cut              │
    └─────────────────────────────────────────┘
                |
                v
             ( End )
```

**FIG. 3**

302

Desired Stiffness Characteristics ← 410

Legend:
- ----o---- Vertical Bending Stiffness (Eiyy) ← 412
- —◇— Chordwise Bending Stiffness (Elzz) ← 414
- --□-- Torsional Stiffness (GJ) ← 416

Logarithmic Stiffness

Normalized Wing Station ← 420

0.1  0.2  0.3  0.4  0.5  0.6  0.7  0.8  0.9  1

FIG. 4

EP 4 332 819 A1

18

CUT 01 (ETA 0.109)
CUT 02 (ETA 0.132)
CUT 03 (ETA 0.148)
CUT 04 (ETA 0.164)
CUT 05 (ETA 0.180)
CUT 06 (ETA 0.196)
CUT 07 (ETA 0.212)
CUT 08 (ETA 0.228)
CUT 09 (ETA 0.244)
CUT 10 (ETA 0.260)
CUT 11 (ETA 0.278)
CUT 12 (ETA 0.295)
CUT 13 (ETA 0.313)
CUT 14 (ETA 0.330)
CUT 15 (ETA 0.348)
CUT 16 (ETA 0.365)
CUT 17 (ETA 0.383)
CUT 18 (ETA 0.401)
CUT 19 (ETA 0.419)
CUT 20 (ETA 0.436)
CUT 21 (ETA 0.456)
CUT 22 (ETA 0.478)
CUT 23 (ETA 0.499)
CUT 24 (ETA 0.520)
CUT 25 (ETA 0.542)
CUT 26 (ETA 0.563)
CUT 27 (ETA 0.584)
CUT 28 (ETA 0.606)
CUT 29 (ETA 0.627)
CUT 30 (ETA 0.649)
CUT 31 (ETA 0.670)
CUT 32 (ETA 0.691)
CUT 33 (ETA 0.713)
CUT 34 (ETA 0.734)
CUT 35 (ETA 0.756)
CUT 36 (ETA 0.777)
CUT 37 (ETA 0.798)
CUT 38 (ETA 0.819)
CUT 39 (ETA 0.841)
CUT 40 (ETA 0.862)
CUT 41 (ETA 0.883)
CUT 42 (ETA 0.904)
CUT 43 (ETA 0.926)
CUT 44 (ETA 0.948)
CUT 45 (ETA 0.970)
CUT 46 (ETA 0.992)

304

FIG. 5

314

602 — Any cuts remaining ?

Yes

604 — Identify variables associated with next cut

606 — Determine bounds for variables

608 — Determine initial guess for variables

610 — Solve nonlinear least squares optimization problem

612 — Set the solve status to true for the variables solved by processing this cut

No

Stop

FIG. 6

FIG. 7

EP 4 332 819 A1

FIG. 8

Europäisches
Patentamt

European
Patent Office

Office européen
des brevets

## EUROPEAN SEARCH REPORT

Application Number

EP 23 17 8787

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DABABNEH ODEH ET AL: "Influence of high fidelity structural models on the predicted mass of aircraft wing using design optimization", AEROSPACE SCIENCE AND TECHNOLOGY, ELSEVIER MASSON, FR, vol. 79, 26 May 2018 (2018-05-26), pages 164-173, XP085429923, ISSN: 1270-9638, DOI: 10.1016/J.AST.2018.05.043 * abstract * * section I, par. 1 section 4.1, par. 1 section 4.2, par. 1 section 4.2, numbered list item 4 section 4.3, par. 1 section 4.3, par. 2 section 4.3, first paragraph pg. 168, col. 2, par. 1 pg. 169, col. 2, last paragraph pg. 171, col. 1, last paragraph pg. 171, col. 2, first paragraph * * figures 1, 3-6 * * table 2 * * eq. 7, 10, 11, 12-17 * * the whole document * | 1-15 | INV. G06F30/15 G06F30/23 ADD. G06F113/26 G06F111/06 |
| | ----- -/-- | | |
| | | | TECHNICAL FIELDS SEARCHED (IPC) |
| | | | G06F |

The present search report has been drawn up for all claims

| Place of search | Date of completion of the search | Examiner |
|---|---|---|
| Munich | 11 January 2024 | Brandiska, Pavlina |

EPO FORM 1503 03.82 (P04C01)

# EP 4 332 819 A1

**EUROPEAN SEARCH REPORT**

Application Number

EP 23 17 8787

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WANG ZEXI ET AL: "Aeroelastic and local buckling optimisation of a variable-angle-tow composite wing-box structure", COMPOSITE STRUCTURES, ELSEVIER SCIENCE LTD, GB, vol. 258, 28 October 2020 (2020-10-28), XP086443635, ISSN: 0263-8223, DOI: 10.1016/J.COMPSTRUCT.2020.113201 [retrieved on 2020-10-28] * abstract * * eq. 19, 20 pg. 9, col. 2, par. 1 pg. 13, col. 1, 2nd paragraph section 3, first paragraph section 4.1 , first paragraph section 2.2.1 * * tables 3, 4, 5 * * figures 5a, 7, 11, 15-17 * * the whole document * | 1-15 | |
| X | CN 113 886 967 A (UNIV BEIHANG) 4 January 2022 (2022-01-04) * abstract * * the whole document * | 1-15 | **TECHNICAL FIELDS SEARCHED (IPC)** |
| X | PANETTIERI ENRICO ET AL: "Multi-scale Least-Weight Design of a Wing-Box Through a Global/Local Modelling Approach", JOURNAL OF OPTIMIZATION THEORY AND APPLICATIONS, vol. 187, no. 3, 25 June 2020 (2020-06-25) , pages 776-799, XP037309007, ISSN: 0022-3239, DOI: 10.1007/S10957-020-01693-Y * abstract * * the whole document * | 1-15 | |

The present search report has been drawn up for all claims

| Place of search | Date of completion of the search | Examiner |
|---|---|---|
| Munich | 11 January 2024 | Brandiska, Pavlina |

EPO FORM 1503 03.82 (P04C01)

**page 2 of 2**

## ANNEX TO THE EUROPEAN SEARCH REPORT
## ON EUROPEAN PATENT APPLICATION NO.

EP 23 17 8787

This annex lists the patent family members relating to the patent documents cited in the above-mentioned European search report.
The members are as contained in the European Patent Office EDP file on
The European Patent Office is in no way liable for these particulars which are merely given for the purpose of information.

11-01-2024

| Patent document cited in search report | | Publication date | Patent family member(s) | Publication date |
|---|---|---|---|---|
| CN 113886967 | A | 04-01-2022 | NONE | |

EPO FORM P0459